# FASCICULE DE BREVET EUROPEEN

(11) **EP 4 138 793 B1**
(45) Date de publication et mention de la délivrance du brevet: **02.07.2025**
(21) Numéro de dépôt: 21719616.1
(22) Date de dépôt: 16.04.2021
(51) Int. Cl.: A61K 31/045, A61P 31/00, A61P 31/04, A61P 31/12, A61P 31/14, A61P 35/00

(54) **NOUVEL INHIBITEUR NON PROTÉIQUE DE FURINE**
NEUER NICHT-PROTEINISCHER FURIN-INHIBITOR
NOVEL NON-PROTEINIC FURIN INHIBITOR

(30) Priorité: 19.04.2020 FR 2003923
(43) Date de publication de la demande: 01.03.2023
(73) Titulaire: Uption, 1725 Luxembourg (LU)
(72) Inventeur: BOURGETEAU, Vincent, 56130 FEREL (FR)
(74) Mandataire: Eveillard, Sophie
(86) Numéro de dépôt international: PCT/EP2021/059960
(87) Numéro de publication internationale: WO 2021/213931

(56) Documents cités:
- WO-A1-2019/055119
- CN-B- 102 335 223
- MAURYA DHARMENDRA KUMAR ET AL: "Evaluation of Traditional Ayurvedic Preparation for Prevention and Management of the Novel Coronavirus (SARS-CoV-2) Using Molecular Docking Approach", 14 April 2020 (2020-04-14), XP055814455, Retrieved from the Internet <URL:https://ayushnext.ayush.gov.in/research/uploads/5fd876dc96315_prevention.pdf> [retrieved on 20210616], DOI: 10.26434/chemrxiv.12110214.v1
- KHWAZA VUYOLWETHU ET AL: "Antiviral Activities of Oleanolic Acid and Its Analogues", MOLECULES, vol. 23, no. 9, 9 September 2018 (2018-09-09), pages 2300, XP055814447, DOI: 10.3390/molecules23092300
- PARVEZ MOHAMMAD K. ET AL: "Analysis of antioxidative and antiviral biomarkers [beta]-amyrin, [beta]-sitosterol, lupeol, ursolic acid in Guiera senegalensis leaves extract by validated HPTLC methods", SAUDI PHARMACEUTICAL JOURNAL, vol. 26, no. 5, 1 July 2018 (2018-07-01), AMSTERDAM, NL, pages 685 - 693, XP055814566, ISSN: 1319-0164, DOI: 10.1016/j.jsps.2018.02.022
- ZHENG XIASHENG ET AL: "Triterpenoid Saponin Biosynthetic Pathway Profiling and Candidate Gene Mining of the Ilex asprella Root Using RNA-Seq", INTERNATIONAL JOURNAL OF MOLECULAR SCIENCES, vol. 15, no. 4, 1 January 2014 (2014-01-01), pages 5970 - 5987, XP055814542, Retrieved from the Internet <URL:https://www.ncbi.nlm.nih.gov/pmc/articles/PMC4013608/pdf/ijms-15-05970.pdf> DOI: 10.3390/ijms15045970
- KADIOGLU ONAT ET AL: "Identification of novel compounds against three targets of SARS CoV-2 coronavirus by combined virtual screening and supervised machine learning", WORLD HEALTH ORGANIZATION, 21 March 2020 (2020-03-21), XP055814602, Retrieved from the Internet <URL:https://www.who.int/bulletin/online_first/20-255943.pdf> [retrieved on 20210616], DOI: 10.2471/BLT.20.255943
- CHOI JI WON ET AL: "Antibacterial Activity of Triterpenoids from Clerodendron trichotomum", JOURNAL OF APPLIED BIOLOGICAL CHEMISTRY, vol. 55, no. 3, 30 September 2012 (2012-09-30), KR, pages 169 - 172, XP055814135, ISSN: 1976-0442, Retrieved from the Internet <URL:https://www.koreascience.or.kr/article/JAKO201235540449824.pdf> DOI: 10.3839/jabc.2012.026
- BRAUN ELISABETH ET AL: "Furin-mediated protein processing in infectious diseases and cancer", CLINICAL & TRANSLATIONAL IMMUNOLOGY, vol. 8, no. 8, 1 January 2019 (2019-01-01), GB, XP055772629, ISSN: 2050-0068, Retrieved from the Internet <URL:https://onlinelibrary.wiley.com/doi/full-xml/10.1002/cti2.1073> DOI: 10.1002/cti2.1073
- NOGUEIRA AMAURILIO O. ET AL: "Pharmacological effects of the isomeric mixture of alpha and beta amyrin from Protium heptaphyllum : a literature review", FUNDAMENTAL & CLINICAL PHARMACOLOGY., vol. 33, no. 1, 5 August 2018 (2018-08-05), FR, pages 4 - 12, XP055814131, ISSN: 0767-3981, Retrieved from the Internet <URL:https://api.wiley.com/onlinelibrary/tdm/v1/articles/10.1111%2Ffcp.12402> DOI: 10.1111/fcp.12402
- MAURYA VIMAL K. ET AL: "Antiviral activity of traditional medicinal plants from Ayurveda against SARS-CoV-2 infection", JOURNAL OF BIOMOLECULAR STRUCTURE & DYNAMICS, 19 October 2020 (2020-10-19), US, pages 1 - 17, XP055814463, ISSN: 0739-1102, Retrieved from the Internet <URL:https://www.tandfonline.com/doi/pdf/10.1080/07391102.2020.1832577?needAccess=true> DOI: 10.1080/07391102.2020.1832577

## Description

Le génome humain code pour plus de 550 ciseaux moléculaires de type protéases. Ces ciseaux moléculaires digèrent les protéines de nos aliments, dégradent les protéines non conformes ou indésirables, elles régulent également le trafic et l'activité de nombreux facteurs cellulaires. Le clivage protéolytique est l'une des modifications post-traductionnelles majeures dans l'homéostasie. Il régule de nombreux processus qui maintiennent la santé ou entraînent la maladie générant un grand nombre de protéines et de peptides bioactifs avec des rôles clés dans la prolifération cellulaire, l'immunité et l'inflammation (Braun E et al., 2019).

Parmi ces ciseaux moléculaires, l'un est ubiquitaire chez les mammifères, il s'agit de la furine, une protéase exprimée de manière omniprésente dans de nombreux tissus cellulaires. Parmi les substrats clivés par la furine chez les mammifères, nous trouvons les cytokines, les hormones, les facteurs de croissance et de nombreux récepteurs. Il est prouvé que la dérégulation de la furine peut déclencher des maladies infectieuses ainsi que le cancer. L'activité enzymatique de la furine est utilisée par de nombreux agents pathogènes viraux et bactériens, afin d'améliorer leur virulence et leur propagation (Braun E et al., 2019).

En effet, la furine joue un rôle clé dans la maturation et la pathogénicité des glycoprotéines virales. Chez les mammifères, elle clive et active un grand nombre de substrats viraux et bactériens. Parmi ces substrats, nous avons les glycoprotéines d'enveloppe virale et les toxines bactériennes, mais aussi des facteurs cellulaires qui favorisent le développement et la croissance des tumeurs s'ils sont hyperactivés (Tian S et al., 2011).

Dans l'évolution, la furine fait partie de l'ancienne famille des pro-protéines convertases. Leur similitude avec la subtilisine bactérienne et les protéases de la levure kexine est à l'origine de l'abréviation PCSK (Proprotein Convertase Subtilisin / Kexin type). Les humains codent pour neuf membres de cette famille de protéases (PCSK1 à 9), PCSK3 représentant la furine. Les PCSK sont bien connues pour leur capacité à activer d'autres protéines cellulaires (Braun E et al., 2019).

À ce jour, plus de 200 substrats cellulaires de PCSK ont été décrits, y compris des hormones, des récepteurs, des facteurs de croissance et des molécules d'adhésion (Roeabroek A et al., 1986 ; Van de ven WJ et al., 1990).

Les PCSK1 à 7, notamment la furine (PCSK3) clivent leurs substrats après une séquences de résidus basiques, avec un motif de reconnaissance typique (Seidah NG et al., 2012) PCSK3 clive des motifs basiques d'acides aminés, c'est pourquoi il a été appelé PACE (enzyme de clivage basique des acides aminés). La furine est exprimée par la FUR (FES Upstream Region) un gène porté sur le chromosome 15.

Des niveaux élevés de furine se trouvent dans les glandes salivaires, le foie et la moelle osseuse, alors que les cellules musculaires en expriment des quantités relativement faibles.

Lors de la traduction de l'ARNm, la furine entre dans la voie de sécrétion en tant que proenzyme inactive et est intégrée dans la membrane RE (Reticulum Endoplasmique) *via* son domaine transmembranaire C-terminal. Comme chez de nombreuses protéines transmembranaires, elle abrite un court peptide signal N-terminal qui est clivé par co-translation. Semblable à d'autres pro-protéines convertases, la furine contient un pro-peptide inhibiteur de 83 acides aminés N-terminal, dont la fonction de chaperon est requise pour un repliement correct du domaine catalytique (Shinde U *et al.,* 1993). Le propeptide inhibiteur en position N teminale est éliminé dans un processus d'auto-protéolyse en deux étapes, et c'est à alors que la furine devient une enzyme active (Anderson ED, 1997). En même temps, des oligosaccharides liés à N sont ajoutés et coupés. Bien que la furine s'accumule dans l'appareil de Golgi (compartiment TGN), elle peut être transportée vers la surface cellulaire et retour *via* la voie endosomale (Pesu M et al., 2006 ; Blanchette F et al., 1997 ; Decroly E et al., 1994 ; Hipp MM et al., 2013).

La furine peut également être éliminée et libérée dans l'espace extracellulaire lors de la séparation par clivage protéique du domaine catalytique de l'extrémité C liée à la membrane. Il reste à déterminer si cette étape de clivage est médiée par la furine elle-même ou par une autre protéase (Vey M et al., 1994). La présence de furine dans l'appareil de Golgi au niveau du TGN et les compartiments endosomaux, à la surface des cellules et dans l'espace extracellulaire, peuvent expliquer sa capacité à traiter une grande variété de substrats intra et extracellulaires (Plaimauer B et al., 2001).

Des analyses bio-informatiques et des études fonctionnelles ont révélé, chez les mammifères, plus de 100 sites de clivage de la furine (Tian S, 2009). Ceux-ci comprennent notamment des facteurs de croissance et des cytokines (par exemple IGF1, IGF2, TGFb, PDGFa, PDGFb, VEGF-C, NGF, CXCL10), des hormones (par exemple PTH, TRH, GHRH), des molécules d'adhésion (par exemple intégrines, vitronectine), collagènes, métalloprotéinases, facteurs de coagulation, récepteurs, canaux membranaires et albumine.

La furine est désormais au cœur de la lutte contre le cancer.

La furine a été qualifiée d'amorce majeure et de médiateur clé dans la croissance et la progression tumorale. En effet, son expression ou son activation excessive peut favoriser la formation et la progression de diverses tumeurs malignes, notamment le carcinome du côlon, le rhabdomyosarcome, les cancers de la tête et du cou, les tumeurs du poumon, de la peau et du cerveau (Jaaks P et al., 2017). Souvent, les niveaux de furine sont en corrélation positive avec l'agressivité cancéreuse, c'est pourquoi une augmentation de l'expression de la furine a été proposée comme marqueur pronostique des cancers avancés (Bassi DE et al., 2005 ; Klein Szanto AJ et al., 2017 ; Jaaks P et al., 2017).

L'activité oncogène de la furine a été attribuée à sa capacité à activer des protéines qui favorisent la prolifération cellulaire, l'angiogenèse, la migration et l'invasion tissulaire. Par exemple, la furine clive et active des facteurs de croissance tels que les IGF, PDGF ou NGF qui améliorent la prolifération cellulaire et par conséquent la croissance tumorale.

L'expression de la furine est induite par l'hypoxie, car les trois promoteurs FUR abritent des sites de liaison pour le *Facteur 1* inductible par l'hypoxie (HIF-1) (McMahon S et al., 2005).

Ainsi, une vascularisation médiée par la furine peut se produire préférentiellement dans des tumeurs hypoxiques à croissance restreinte. Fait aggravant, l'hypoxie entraîne une relocalisation subcellulaire de la furine à la surface des cellules, ce qui favorise encore le traitement des facteurs de croissance et d'autres tumorigènes extracellulaires à protéines précurseurs (Arsenault D et al., 2012).

Outre les effets sur la croissance tumorale, la furine favorise la migration et l'extravasation des cellules malignes car la furine traite les molécules d'adhésion médiatrices des interactions cellule-cellule et cellule-matrice. Le clivage des intégrines peut être particulièrement pertinent car au-delà de la médiation de l'adhésion des cellules à la matrice extracellulaire, elles agissent également comme des transducteurs de signaux régulant la croissance, la division et la survie des cellules (Stupack DG et al., 2002).

Il est à noter en facteur aggravant que la furine active les métalloprotéinases matricielles (par exemple MMP14) qui facilitent la progression des métastases en dégradant les composants de la matrice extracellulaire (Jaaks P et al., 2017). L'augmentation de l'activité de la furine favorise le développement du cancer en supprimant les mécanismes antitumoraux protecteurs ; à titre d'exemple, il est désormais connu que l'augmentation de l'activation médiée par la furine du TGFb réduit la surveillance immunitaire en favorisant le développement de cellules Treg suppressives et en inhibant les fonctions effectrices des lymphocytes T (Dahmani A et al., 2018).

De manière indirecte, les boucles de rétroaction positive peuvent encore accroître le potentiel oncogène de la furine; à titre d'exemple, le substrat de furine TGFb augmente non seulement l'expression de l'ARNm de la furine, mais ce substrat améliore également l'activité protéolytique de la furine par un mécanisme encore inconnu (Bourne GL et al., 2011).

De même, la furine augmente la sécrétion de cytokines (IFNc), qui à leur tour activent le promoteur FUR favorisant ainsi la transcription du gène codant pour la furine (Pesu M et al., 2006 ; Hipp MM, 2013).

Cette amélioration mutuelle semble particulièrement critique étant donné le rôle clé de l'IFNc dans le développement et la progression de la tumeur (Mojic M et al., 2017).

D'une part, la libération d'IFNc entraînée par la furine peut avoir des effets bénéfiques car elle stimule l'activité anti-tumorale des cellules tueuses naturelles et des lymphocytes T cytotoxiques. En outre, l'IFNc peut agir comme un facteur anti-angiogénique et inhiber directement la prolifération des cellules tumorales en induisant l'expression de suppresseurs de tumeurs tels que P21 ou P27. D'un autre côté, cependant, des preuves récentes suggèrent que l'IFNc peut favoriser la tumeur, par exemple en sélectionnant des phénotypes immuno-évasifs et en favorisant un microenvironnement tumoral immunosuppresseur (Mojic M et al., 2017).

Curieusement, une étude sur des patients atteints de cancer du larynx suggère que la boucle de rétroaction IFNc-furine pourrait être encore renforcée par voie iatrogène puisque la radiothérapie a augmenté l'expression de la furine chez certains patients (Lee M et al., 2016).

En résumé, l'activation excessive de la furine favorise plusieurs étapes du développement du cancer, y compris la prolifération cellulaire, la vascularisation, les métastases et l'immunité antitumorale.

Un inhibiteur de l'activité furine peut donc jouer un rôle clé pour enrailler le développement du cancer, notamment pour éviter la prolifération cellulaire, éviter les métastases, restreindre la vascularisation tumorale, et améliorer l'immunité anti-tumorale.

Un inhibiteur de l'activité furine peut également limiter les effets secondaires de la radiothérapie et augmenter le rapport bénéfice/risque de ce mode thérapeutique.

La furine est capable d'activer par protéolyse une grande variété de protéines dérivées d'agents pathogènes.

En particulier chez les bactéries, le groupe de toxines AB comprend plusieurs substrats de furine bien décrits. Ces exotoxines sont sécrétées par des bactéries et exercent leur effet dans le cytoplasme de la cellule cible. Elles se composent généralement d'une sous-unité A qui porte l'activité enzymatique ou toxique et d'une sous-unité B qui assure la liaison et la translocation avec la membrane. Pour exercer son effet toxique, la sous-unité A doit être séparée de la sous-unité B associée à la membrane par clivage protéolytique (Gordon VM et al., 1994).

Avec le cas de la toxine diphtérique (issue de *Corynebacterium diphtheriae* ou *bacille de Löffler-Klebs*) ou de l'exotoxine A de *Pseudomonas aeruginosa,* toxine étudiée dans le cadre du traitement de l'hépatite B (Hafkemeyer P, 2008 DOI: 10.3748/wjg.14.2810), le clivage se produit très probablement dans les endosomes avant que la sous-unité A ne se déplace dans le noyau où elle inhibe la synthèse des protéines en inhibant le facteur d'élongation EF2 (Collier RJ, 1967).

Avec le cas de la toxine de l'anthrax (issue de *Bacillus anthracis*), une exotoxine à trois protéines constituées de l'antigène protecteur de liaison aux récepteurs (PA) et du facteur œdème (EF) et du facteur létal (LF), le schéma de clivage diffère. En effet, lors de la liaison à son récepteur, le PA est clivé par la furine à la surface des cellules. Cette étape de clivage déclenche l'oligomérisation du PA en un complexe qui lie EF et LF. Par la suite, ce complexe de toxines est endocyté et l'AP forme un canal qui permet la translocation d'EF et de LF dans le cytoplasme (Gordon VM et al., 1994). Bien que le PA puisse être activé par différents membres de la famille des proprotéines convertases, la furine semble être la principale toxine de l'anthrax activant la protéase toxique (Molloy SS et al., 1992).

Ces exemples illustrent que plusieurs agents pathogènes bactériens exploitent la furine et les convertases apparentées pour l'activation de leurs exotoxines. À strictement parler, cependant, certaines toxines produites par des bactéries (par exemple la toxine diphtérique) représentent des produits de gènes viraux car elles sont codées par des bactériophages (Wagner PL et al., 2002). Dans ces cas, le terme « exotoxine virale » est plus approprié. Cela suggère fortement que l'activation de la toxine médiée par la furine confère un avantage de sélection à la fois à la bactérie et à son phage.

Par exemple, l'induction de la mort cellulaire par des toxines activées par la furine favorise l'invasion tissulaire, elle augmente les taux de transmission (par exemple en provoquant la diarrhée) ou supprime les réponses immunitaires cellulaires. Sans l'activation protéolytique des exotoxines, des maladies telles que la dysenterie ou la diphtérie ne se produiraient pas.

Un inhibiteur de l'activité furine pourra donc éviter l'activation de nombreuses toxines bactériennes (elles-mêmes, éventuellement d'origines virales), notamment le groupe des toxines AB avec un inhibiteur intracellulaire qui maintiendra les toxines passives et sans virulence. Un inhibiteur de l'activité furine pourra également stopper et esquiver dans le milieu extra-cellulaire des toxines telles que l'anthrax.

Dans la lutte contre les toxines bactériennes et pour leur neutralisation, les inhibiteurs de furine sont des substances à intégrer dans la prévention et le traitement des maladies infectieuses impliquants ces toxines.

Comme les exotoxines bactériennes et virales, la plupart des glycoprotéines d'enveloppe virale doivent être clivées par protéolyse avant de pouvoir médier l'entrée virale dans les cellules hôtes. Dans de nombreux cas, les virus exploitent à cette fin des endoprotéases cellulaires de type trypsine ou subtilisine. Alors que les protéases de type subtilisine telles que la furine nécessitent des sites de clivage polybasiques, les protéases de type trypsine reconnaissent également les motifs monobasiques et se clivent après les résidus d'arginine ou de lysine simples (Klenk HD et al., 1994 https://doi.org/10.1016/0966-842X(94)90123-6).

Il est à noter que la dépendance à l'égard de protéases spécifiques est un point clé du tropisme tissulaire et de la propagation virale dans un organisme infecté. Par exemple, les souches avirulentes du virus de la maladie de Newcastle (NDV) abritent un site de clivage monobasique dans leur protéine Fusion (F) et n'entraînent que des infections locales (principalement dans les voies respiratoires) car l'expression des protéases hôtes respectives est limitée à quelques types de cellules. En revanche, les protéines F des souches virulentes de NDV peuvent être clivées par la furine. Par conséquent, ces virus peuvent se propager de façon systémique et provoquer des taux de mortalité élevés chez les oiseaux infectés ( Nagai Y et al., 1989). Un autre exemple bien décrit est le clivage de l'hémagglutinine (HA) du virus de la grippe A. Contrairement aux virus de l'influenza aviaire A faiblement pathogènes, leurs homologues hautement pathogènes hébergent un site de clivage de la furine polybasique dans la protéine HA48. Ainsi, la capacité des virus à exploiter la furine peut avoir des effets déterminants sur leur pathogénicité.

À ce jour, le clivage protéolytique de la furine est connu et opérationnel sur les glycoprotéines d'enveloppe codées par de nombreuses familles de virus diverses, notamment les virus Herpès, Coronavirus, Flavivirus, Togavirus, Bornavirus, Bunyavirus, Filovirus, Orthomyxovirus, Paramyxovirus, Pneumo and Retroviridae. Les protéines d'enveloppe de certains virus sont clivées dans la cellule productrice, d'autres sont traitées dans l'espace extracellulaire ou lors de l'entrée dans leurs cellules cibles.

Dans le cas des trimères rétroviraux de glycoprotéines, tels que ceux de l'immunodéficience humaine, du sarcome de Rous ou des virus de la leucémie murine, sont traités et activés par clivage protéique dans les cellules productrices.

La furine joue un rôle particulièrement important dans l'infection et la pathogénicité Virus d'Immunodéficience Humaine (VIH).

En effet, dans le cas du VIH-1, le précurseur gp160 de la protéine d'enveloppe virale (Env) est clivé en gp120 et en gp41 ancré dans la membrane qui restent associés par des interactions non covalentes. Le clivage protéolytique de la furine se produit dans les compartiments intracellulaires, avant l'assemblage des virions au niveau de la membrane plasmique. Il est à noter que le traitement protéolytique de Env dépend d'une glycosylation liée à N correcte, car les chaînes latérales aberrantes des glucides peuvent entraîner une erreur de traitement sous-cellulaire ou la séquestration de Env (Moulard M et al., 2000). Très probablement, le VIH-1 profite de la redondance de plusieurs proprotéines convertases reconnaissant le motif de clivage polybasique dans Env. Furin, PCSK5, PCSK6 et PCSK7 se sont tous révélés cliver la gp160 dans les cellules, bien qu'avec des efficacités différentes (Moulard M et al., 2000).

Dans le cas des hémagglutinines (HA) du virus de la grippe A, il est à noter que les hémagglutinines H5 et H7 d'un grand nombre de virus de l'influenza aviaire A hautement pathogènes (HPAIV) peuvent être clivées par la furine ou PCSK5, qui sont présentes dans de nombreux types de cellules (Stieneke Gröber A et al., 1992 ; Horimoto T et al., 1994).

Il est à noter que la capacité d'exploiter la furine pour un clivage efficace de HA et l'augmentation associée de la pathogénicité, sont déterminées par la présence d'un site cible consensus de furine.

La furine clive les glycoprotéines (GP) du virus de Marburg (MARV) et les cinq espèces d'Ebolavirus en une grande sous-unité N-terminale (GP1) qui assure la liaison du récepteur et une petite partie C-terminale ancrée à la membrane (GP2) qui contient le peptide de fusion (Volchkov VE et al., 1998 ; Volchkov VE et al., 2000).

Ainsi, la conservation élevée du site de clivage de la furine parmi les différentes espèces d'Ebolavirus est marquante et il reste à déterminer si le traitement GP médié par la furine joue un rôle dans le réservoir naturel hôtes de ces virus (Wooi-Lewis RJ et al., 1999).

Notamment, le gène EBOV GP abrite une séquence de site d'édition d'ARN et peut non seulement exprimer la GP de pleine longueur, mais aussi une forme soluble de la glycoprotéine (pré-sGP) qui n'a pas le domaine transmembranaire C-terminal Volchkov VE et al., 1995).

Curieusement, le pré-sGP en abrite un autre site de reconnaissance de la furine et est clivé en sGP mature et un soi-disant peptide D court. Les deux sont finalement libérés des cellules infectées (Volchkova VA et al., 1999). Entre autres, le sGP est supposé servir d'antigène leurre, pour agir comme un substitut structurel de GP1 et pour induire l'apoptose des lymphocytes non infectés (De la Vega MA et al., 2015).

Dans le cas des Flavivirus, l'ARN du flavivirus est traduit en une seule grande polyprotéine qui est clivée par des protéases cellulaires et virales en toutes les protéines structurales et non structurales du virus. Les protéines structurales comprennent les protéines d'enveloppe prM et E qui sont incorporées en tant qu'hétérodimères prM / E dans les virions en herbe.70 prM agit comme un chaperon et facilite le repliement correct de la glycoprotéine E (Guirakhoo F et al., 1991).

De nombreux flavivirus bourgeonnent dans la lumière du Reticulum Endoplasmique (RE) et entrent dans la voie de sécrétion (Pierson TC et al., 2012). Dans le réseau trans-Golgi (TGN), un site de clivage de la furine est exposé et prM peut être clivé en protéines pr et M matures (Yu IM et al., 2008). Ainsi, le clivage médié par la furine de la glycoprotéine virale ne se produit qu'après son incorporation dans des virions nouvellement formés. Le peptide pr reste associé à la protéine E jusqu'à ce que le virion soit libéré de la cellule, empêchant ainsi une fusion prématurée involontaire avec les membranes de la cellule productrice (Yu IM et al., 2009). Le clivage prM médié par la furine est essentiel pour la réplication des flavivirus tels que l'encéphalite à tiques ou les virus de la dengue (Elshuber S et al., 2003 ; Zybert EA et al., 2008).

Chez les virus de la dengue de grandes quantités de virus immatures ou partiellement matures sont libérés, très probablement en raison d'un résidu acide (défaut) conservé dans le site de reconnaissance de la furine (Junjhon J et al., 2008). Ainsi la furine se montre déterminante dans la maturité et la virulence des virus de la dengue. Il est aussi important de noter que la teneur en prM dans les particules virales affecte également la reconnaissance des anticorps et, par conséquent, la stimulation dépendante des anticorps de l'infection par le virus de la dengue (Rodenhuis-Zybert IA et al., 2010).

Par conséquent, chez notamment les virus de la dengue, l'inhibition de la furine permet de préserver le complexe protéique prM ce qui favorise le bon fonctionnement de la réaction immunitaire de défense de l'hôte.

Alors que la furine joue un rôle clé dans l'activation des glycoprotéines d'enveloppe d'une variété de virus, son activité est également exploitée pour le clivage d'autres protéines virales. Le clivage de la protéine L2 des papillomavirus en est une illustration (Richards RM et al., 2006).

L'ensemble, constitué par la protéine de capside majeure L1 et cette protéine L2 de capside mineure, construit la capside virale. Le site de clivage de la furine est situé près de l'extrémité N de L2 de la capside, il est hautement conservé parmi différentes souches de papillomavirus humain (HPV) (Day PM et al., 2009). Le clivage n'est pas requis pour l'assemblage ou la libération du virus, mais est essentiel pour l'infection de nouvelles cellules cibles.

Le papillomavirus L2 est exclusivement clivé sur les cellules cibles/ hôtes (Day PM et al., 2009).

Ainsi, il apparaît que les virus non enveloppés ont également développé la capacité d'exploiter la furine notamment dans leur stratégie de prolifération sur de nouvelles cellules hôtes.

Une autre protéine n'appartenant pas à une enveloppe virale est clivée par la furine, il s'agit de l'antigène central externe (HBeAg) du virus de l'hépatite B (HBV) (Ito K et al., 2009 ; Messageot F et al., 2003). L'Ag HBe clivé est sécrété par les cellules infectées et exerce des effets immunosuppresseurs (Milich DR et al., 1998). Il a été suggéré d'agir en empêchant la destruction des hépatocytes infectés par les lymphocytes T cytotoxiques (Chen MT et al., 2004). En revanche, l'AgHBe non clivé peut avoir les effets opposés lorsqu'il est transporté vers la membrane plasmique où il peut déclencher des réponses immunitaires antivirales (Schlicht HJ et al., 1989). Ainsi, le clivage médié par la furine de l'AgHBe peut affecter l'issue de l'infection en favorisant la réponse immunitaire.

En 2013, Aerts et ses collègues ont découvert que le récepteur 1 activé par la protéase (PAR1), un récepteur couplé à la protéine G, interfère avec l'expression de la furine et le traitement médié par la furine de la protéine F du métapneumovirus humain (Aerts L et al., 2013). Des expériences de suivi ont révélé que PAR1 abrite un motif qui assure l'interaction avec plusieurs PCSK (Kim W et al., 2015). Dans cette optique, les PC5A / PCSK5 et PCSK6 solubles clivent PAR1 et abrogent sa capacité à induire la signalisation calcique lors d'un clivage médié par la thrombine au niveau de la membrane plasmique. Étonnamment, cependant, les PCSK liés à la membrane comme la furine ne parviennent pas à cliver le PAR1 à cette position. Au lieu de cela, la furine emprisonne PAR1 dans le réseau trans-Golgi et empêche son transport antérograde à la surface cellulaire. En même temps, PAR1 bloque également l'activité protéolytique de la furine, inhibant par exemple la maturation du VIH-1 Env. Cette activité inhibitrice n'est pas partagée par son PAR2 paralogue, qui est efficacement clivé par la furine (Sachan V et al., 2019). Notamment, l'expression de PAR1 est induite dans des environnements pro-inflammatoires tels que le cerveau d'individus infectés par le VIH-1 souffrant de troubles neurocognitifs associés au VIH (MAIN) (Kim W et al., 2015).

Ainsi, l'inhibition de la furine médiée par PAR1 peut représenter un mécanisme d'immunité innée limitant la propagation du VIH-1 et potentiellement d'autres agents pathogènes viraux dépendants de la fourrure. Une activité inhibitrice similaire a été récemment décrite pour deux GTPases inductibles par l'IFNc, appelées protéines de liaison au guanylate 2 et 5 (GBP2 et GBP5). Initialement, GBP5 a été décrit dans un dépistage de nouveaux facteurs de restriction du VIH et a montré qu'il interférait avec la maturation de la protéine Env rétrovirale. En conséquence, le clivage du précurseur Env gp160 en gp120 et gp41 matures est réduit et les virions nouvellement formés ne sont que faiblement infectieux. Étant donné que de nombreux agents pathogènes viraux dépendent de la furine ou des PCSK apparentés pour la maturation de leurs propres protéines (glyco), GBP2 et GBP5 exercent une large activité antivirale, inhibant la réplication de l'influenza aviaire hautement pathogène A, de la rougeole et du virus Zika. En revanche, GBP2 et GBP5 ne diminuent pas l'infectiosité des virions porteurs de la glycoprotéine du virus de la stomatite vésiculaire, qui ne nécessite pas d'étape d'activation protéolytique. Il est à noter que l'inhibition de la furine dans les cellules infectées a un coût, car le traitement médié par la furine des métalloprotéinases matricielles et d'autres substrats cellulaires est également réduit en présence de niveaux accrus de GBP2 ou GBP5 (Braun E et al., 2019). Il est à noter qu'une expression accrue de GBP2 / 5 est associée à des résultats favorables chez les patients souffrant de mélanome ou de cancer du sein (Wang Q et al., 2018 ; Godoy P et al., 2014).

En résumé, chez les virus enveloppés, un inhibiteur de furine peut bloquer l'infection et ou la pathogénécité des virus Herpès, des Coronavirus, des Flavivirus, des Togavirus, des Bornavirus, des Bunyavirus, des Filovirus, des Orthomyxovirus, des Paramyxovirus, des Pneumovirus and Retroviridae, et plus particulièrement chez le Virus d'immunodéficience humaine (VIH), le virus du sarcome de Rous, les virus de la leucémie murine, les virus de l'influenza aviaire A, le virus de Marburg, les cinq espèces d'Ebolavirus, le virus de l'encéphalite à tiques et les virus de la dengue.

En cas d'attaque virale, l'inhibition de la furine peut favoriser le bon fonctionnement de la réaction immunitaire de défense de l'hôte, notamment chez les virus de la dengue.

Chez les virus non enveloppés, un inhibiteur de furine peut bloquer la propagation infectieuse des papillomavirus, et favoriser une bonne réponse immunitaire dans le cade du virus de l'hépatite B.

En raison du rôle clé de la furine dans la pathogenèse du cancer et des maladies infectieuses, son aptitude en tant que cible thérapeutique a suscité un vif intérêt depuis plusieurs années. De nombreux laboratoires ont exploré la possibilité de limiter la croissance tumorale, la réplication virale ou l'intoxication bactérienne en réduisant la quantité ou l'activité protéolytique de la furine.

Initialement, la plupart des études se sont concentrées sur les peptides ou les protéines qui se lient au site actif de la furine et inhibent la liaison au substrat de manière compétitive. Par exemple, une variante de l'inhibiteur de sérine-protéase d'origine naturelle a-1 antitrypsine a été modifiée pour héberger un site consensus de clivage de la furine. Cette variante, appelée a-1 antitrypsine Portland (a1-PDX), inhibe la furine et le PCSK5 et s'est révélée empêcher le traitement du VIH-1 Env et du virus de la rougeole F in vitro (Anderson ED et al., 1993 ; Watanabe M et al., 1995).

L'hémagglutinine et les polyarginines, rivalisent avec les substrats naturels de la furine (Strongin A et al., 2009 ; Cameron A et al., 2000). Même l'ajout exogène du propeptide auto-inhibiteur de la furine a montré qu'il réduisait son activité enzymatique, limitant par exemple l'activation de la MMP9 dans les cellules cancéreuses du sein (Zhong M et al., 1999 ; Lapierre M et al., 2007). Cependant, le potentiel thérapeutique du propeptide n'a jamais été évalué in vivo et les effets inhibiteurs sont très probablement limités car il est connu qu'il se dissocie de la furine dans le TGN (compartiment de l'appareil de Golgi). Plusieurs approches, y compris l'incorporation de D- au lieu de L-acides aminés, ont été appliquées pour augmenter la stabilité et donc l'efficacité des inhibiteurs de la furine. Par exemple, l'hexa-D-arginine (D6R), l'un des premiers inhibiteurs de la furine, présente une bonne stabilité et empêche les effets cytotoxiques de l'exotoxine A de Pseudomonas in vitro et in vivo (Sarac MS et al., 2002). De même, l'application topique de nona-D-arginine (D9R ) réduit les dommages cornéens chez les souris infectées par *Pseudomonas aeruginosa* (Karicerla P et al., 2009). Le D9R a également montré une activité bactéricide directe, probablement en raison de sa nature polycationique (Karicherla P et al., 2010). Outre les acides aminés D, l'incorporation d'analogues d'acides aminés tels que les mimétiques de l'arginine décarboxylée ou Le 4-amidinobenzylamide (Amba) a été utilisé pour augmenter la stabilité des inhibiteurs de la furine dérivés de peptides (Becker GL et al., 2010). De plus, l'ajout d'un fragment chlorométhyl cétone (CMK) à l'extrémité C d'un motif de clivage polybasique s'est avéré utile car il se traduit par alkylation du site actif de la furine qui bloque irréversiblement son activité enzymatique (Henrich S et al., 2003). Néanmoins, la cytotoxicité des inhibiteurs à base de CMK et l'instabilité de la fraction CMK peuvent limiter leur utilisation à des applications topiques telles que le traitement des infections cutanées du VPH (Couture F et al., 2015).

L'élucidation de la structure cristalline de la furine a permis la modélisation ciblée d'inhibiteurs non peptidiques tels que les composés à base de streptamine. Lors de l'ajout de résidus de guanidine, les dérivés de la streptamine imitent le site de clivage de la furine cationique et inhibent son activité enzymatique dans la gamme nanomolaire in vitro (Jiao G-S et al., 2006). Dahms et ses collègues décrivent un exemple intéressant d'inhibiteur dérivé de la 2,5 didéoxystreptamine, où deux molécules de l'inhibiteur forment un complexe avec la furine (Dahms SO et al., 2017). Alors que la première molécule inhibitrice interfère directement avec la conformation de la triade catalytique, la seconde molécule se lie à un tronçon peptidique plan adjacent. Pour inhiber l'activation de la toxine de l'anthrax, l'inhibiteur n'a pas besoin d'entrer dans les cellules, car la furine clive le précurseur de la toxine à la surface des cellules. En revanche, la pénétration de l'inhibiteur dans la cellule est essentielle pour empêcher la maturation du VIH-1 Env et d'autres glycoprotéines virales qui sont clivées par voie intracellulaire. Alors que certains inhibiteurs (par exemple les peptides dérivés de HA) pénètrent efficacement dans les cellules, d'autres ont été modifiés pour augmenter leur disponibilité intracellulaire. Par exemple, l'ajout d'un fragment décanoyle aux inhibiteurs de CMK augmente leur capacité à pénétrer dans les cellules (Garten W et al., 1989).

Alors que de nombreux inhibiteurs décrits ci-dessus réduisent puissamment l'activité de la furine *in vitro* et *in vivo,* la plupart d'entre eux inhibent également d'autres proprotéines convertases reconnaissant les sites de clivage polybasiques identiques ou similaires. Cette limitation est inhérente aux inhibiteurs compétitifs qui visent à imiter la séquence cible de la furine et peut être surmontée par des inhibiteurs allostériques qui se lient à des motifs spécifiques de la furine en dehors du site actif. Un exemple est le nanbody Nb14, qui se lie au domaine C-terminal de la furine, bloquant ainsi l'accès de substrats plus grands au site actif. Notamment, Nb14 se lie spécifiquement au domaine P de la furine et ne reconnaît pas les autres PCSK (Zhu J et al., 2012).

L'inventeur a mis en évidence qu'une famille de composés, les triterpènes pentacycliques, peuvent être utilisés en tant que principes actifs inhibiteurs des PCSK (proprotein convertase subtilisin / kexin type), et plus particulièrement de la PCSK3 aussi appelée furine.

De manière inattendue et inédite, l'inventeur a montré que les triterpènes pentacycliques et plus particulièrement les mélanges d' α et β amyrines, dont la structure chimique est très robuste et dépourvue d'azote, et donc de nature non protéïque peuvent être utilisés en tant qu'inhibiteurs de PCSK, et plus particulièrement la PCSK3.

Ainsi un premier objet de l'invention vise un triterpène pentacyclique choisi parmi les α et β amyrines, pour son utilisation en tant que principe actif inhibiteur de la PCSK3 aussi appelée furine pour le traitement de maladies infectieuses chez les mammifères pour bloquer les toxines microbiologiques de type AB et les maintenir passives et sans virulence, pour stopper l'anthrax, comme anti-viral chez les virus enveloppés, bloquant l'infection et la pathogénécité des Coronavirus, des Flavivirus, des Togavirus, des Bornavirus, des Bunyavirus, des Filovirus, des Paramyxovirus, des Pneumovirus et Retroviridae, et plus particulièrement du Virus d'Immunodéficience Humaine (VIH), du virus du sarcome de Rous, des virus de la leucémie murine, du virus de Marburg, des cinq espèces d'Ebolavirus, du virus de l'encéphalite à tiques et des virus de la dengue, pour favoriser le bon fonctionnement de la réaction immunitaire de défense de l'hôte chez les virus de la dengue, comme anti-viral chez les virus non enveloppés en bloquant la propagation infectieuse des papillomavirus.

Ces dernières années, plusieurs inhibiteurs peptidiques et non peptidiques ont été développés pour bloquer l'activation des toxines bactériennes, empêcher la maturation des protéines virales et supprimer la croissance tumorale.

En particulier l'article « Analysis of antioxidative and antiviral biomarkers β-amyrin, β-sitosterol, lupeol, ursolic acid in Guiera senegalensis leaves extract by validated HPTLC methods » de Parvez et al. Saudi Pharmaceutical Journal vol.26 n°5 pages 685-693 divulgue en page 692, colonne de gauche, premier paragraphe que la β-amyrine est connue pour ses propriétés antivirales contre le virus influenza A et l'article « Triterpenoid Saponin Biosynthetic Pathway Profiling and Candidate Gene Mining of the Ilex asprella Root Using RNA-Seq » de Xiasheng Zheng et al. International Journal of molecular Sciences vol.15 n°4 pages 5970-5987 divulgue en page que *l'Ilex asprella* qui contient une grande quantité d' α-amyrine est une plante médicinale connue pour ses propriétés antivirales contre le virus influenza A. Ces articles ne mentionnent pas les α et β amyrines, pour leur utilisation en tant que principe actif inhibiteur de la furine pour le traitement de maladies infectieuses selon la présente invention.

Le principe actif utilisé selon l'invention est un triterpène pentacyclique, choisi parmi les amyrines purifiées et les extraits naturels contenant des amyrines et dérivés d'amyrines, notamment les extraits de Burséracées, et plus particulièrement les extraits de Protium heptaphyllum.

Avantageusement, ledit triterpène pentacyclique est constitué d'α amyrine (à 100% ou viminalol), ou de β amyrine (à 100%), ou d'un mélange d'α et β amyrines contenant de 20% à 80% d'α amyrine et de 20% à 80% de β amyrine de préférence d'un mélange d'α et β amyrines contenant de 20% à 80% d'α amyrine et de 20% à 80% de β amyrine et de manière préférée un mélange comprenant 60% d'α amyrine et de 40% de β amyrine.

De préférence, le principe actif comprend des α et β amyrines et plus particulièrement des amyrines purifiées de Protium heptaphyllum» qui est un complexe composé de 60% d'α amyrine et de 40% de β amyrine.

Selon une première mise en œuvre de l'invention, le triterpène pentacyclique est utilisé selon l'invention pour bloquer les toxines microbiologiques de type AB et les maintenir passives et sans virulence.

Selon une deuxième mise en œuvre de l'invention, le triterpène pentacyclique est utilisé selon l'invention pour stopper l'anthrax.

Selon une troisième mise en œuvre de l'invention, le triterpène pentacyclique est utilisé selon l'invention comme anti-viral chez les virus enveloppés, bloquant l'infection et la pathogénécité des Coronavirus, des Flavivirus, des Togavirus, des Bornavirus, des Bunyavirus, des Filovirus, des Paramyxovirus, des Pneumovirus et Retroviridae, et plus particulièrement du Virus d'Immunodéficience Humaine (VIH), du virus du sarcome de Rous, des virus de la leucémie murine, du virus de Marburg, des cinq espèces d'Ebolavirus, du virus de l'encéphalite à tiques et des virus de la dengue.

Selon une quatrième mise en œuvre de l'invention, le triterpène pentacyclique est utilisé selon l'invention pour favoriser le bon fonctionnement de la réaction immunitaire de défense de l'hôte, notamment chez les virus de la dengue. Selon une cinquième mise en œuvre de l'invention, le triterpène pentacyclique est utilisé selon l'invention comme anti-viral chez les virus non enveloppés en bloquant la propagation infectieuse des papillomavirus.

L'inhibiteur de l'activité de la furine selon l'invention permet donc d'éviter l'activation de nombreuses toxines bactériennes (elles-mêmes, éventuellement d'origines virales), notamment le groupe des toxines AB avec un inhibiteur intracellulaire qui maintient les toxines passives et sans virulence. L'inhibiteur de l'activité de la furine selon l'invention permet également de stopper et esquiver dans le milieu extra-cellulaire des toxines telles que l'anthrax.

Dans la lutte contre les toxines bactériennes et pour leur neutralisation, les inhibiteurs de l'activité de furine selon l'invention sont des substances à intégrer dans la prévention et le traitement des maladies infectieuses impliquants ces toxines.

Selon l'invention, l'utilisation des α et β amyrines en tant qu'inhibiteurs de l'activité de la furine, chez les virus enveloppés, bloque ou participe à bloquer l'infection et la pathogénécité des Coronavirus, des Flavivirus, des Togavirus, des Bornavirus, des Bunyavirus, des Filovirus, des Paramyxovirus, des Pneumovirus et Retroviridae, et plus particulièrement du Virus d'Immunodéficience Humaine (VIH), du virus du sarcome de Rous, des virus de la leucémie murine, du virus de Marburg, des cinq espèces d'Ebolavirus, de l'encéphalite à tiques et des virus de la dengue.

En cas d'attaque virale, l'utilisation des α et β amyrines en tant qu'inhibiteur de l'activité furine, favorise le bon fonctionnement de la réaction immunitaire de défense de l'hôte, notamment avec les virus de la dengue.

Chez les virus non enveloppés, l'utilisation des α et β amyrines selon l'invention en tant qu'inhibiteur de l'activité furine, bloque et favorise l'arrêt de la propagation infectieuse des papillomavirus. L'utilisation des α et β amyrines selon l'invention est particulièrement mise en œuvre selon des applications thérapeutiques locales, chez les mammifères et plus particulièrement chez l'Homme, telles que le traitement topique des infections bactériennes et virales. Les inhibiteurs de PCSK, et plus particulièrement la PCSK3 sont encore rares et au cœur des stratégies thérapeutiques de pointes dans les domaines évoqués ci-dessus. L'un des principaux défauts des inhibiteurs classiquement utilisés est leur fragilité et capacité à se dégrader rapidement dans les milieux biologiques à cause de leur nature peptidique. En effet, les inhibiteurs classiques sont principalement de nature peptidique même dans le cas de la stratégie d'inhibition allostérique. Les inhibiteurs non peptidiques sont *a priori* un peu plus robustes mais restent structurés avec une fonction amine ou amide (4-amidinobenzylamide, streptamine, 2,5 didéoxystreptamine) qui les rendent mimétiques aux bases aminées des peptides.

L'α et la β Amyrine sont les composants dont l'activité de modulation de l'activité furine est plus particulièrement au cœur de l'invention.

Selon Vasquez (Vasquez LH et al., 2012), les triterpènes pentacycliques sont ubiquitaires dans le règne végétal, dans leurs formes aglycones libres ou dans leurs formes combinées. Les α et β amyrines et leurs analogues sont très communes dans la famille des Burséracées et plus particulièrement dans le genre Protium. La structure chimique de α amyrine est C₃₀H₅₀O, son point de fusion est entre 184°C et 186°C (Sirat, et al., 2010) ; la structure chimique de β amyrine est C₃₀H₅₀O, son point de fusion est entre 189°C et 191°C (Lin et al., 2011).

L'α amyrine est un triterpène d'origine naturelle isolé de diverses sources, notamment des résines végétales. Les quantités les plus importantes de ce triterpène sont disponibles dans les résines de la famille des *Burseraceae* et notamment dans le genre *Protium.* D'autres sources connues d'α amyrine comprennent le copal mexicain (5 g / kg) *Bursera glabrifolia (Burseraceae)* (Hernández-Vázquez, et al., 2010), Cassia obtusifolia (Caesalpinaceae) (140 mg / kg) (Sob et al., 2010) et la résine de *Commiphora holtziana* (syn*. Commiphora erythraea*) *(Burseraceae)* (200 mg / kg) (Manguro et al., 2009).

Les sources les plus importantes de β amyrine comprennent notamment le pollen d'abeille de lotus *Nelumbo nucifera (Nelumbonaceae)* (3 g / kg) (Xu. Et al., 2011), l'écorce de «cuachalalate» *Amphipterygium adstringens (Anacardiaceae)* (2,4 g / kg) (Rosas -Acevedo et al., 2011), isolement de la résine de divers *Protium sp (Burseraceae)* (Alpha-amyrine 1g / kg et Beta-amyrin 1,7 g / Kg) (Dias et al., 2011), résidus de biomasse *d'Eucalyptus globulus (Myrtaceae)* de l'industrie de la pâte à papier (326 mg / kg) (Domingues et al., 2010), latex *Ficus carica (Moraceae)* (1,2 g / kg) (Oliveira et al., 2010), écorce de racine de *Ficus cordata (Moraceae)* (20 mg / kg), écorce de vapeur *Ficus cordata* (200 mg / kg) (Kuete et al., 2008) et les feuilles et l'écorce de *Byrsonima crassifolia* ou *crassa (Malpighiaceae)* (1,3 g / kg) (Higuchi et al., 2008). Des mélanges d'α et de β amyrine ont été obtenus à partir de résidus d'écorce de vapeur de *Byrsonima crassifolia (Malpighiaceae)* (9 g / kg) (Hernández-Vázquez et al., 2010), des feuilles de *Byrsonima fagifolia (Malpighiaceae) (2,3* g / Kg) (Higuchi et al., 2008) et les feuilles de *Pouteria sp (Sapotaceae)* ont donné de l'α, de la β amyrine et d'autres triterpènes (Silva et al., 2009). Ci-dessous une liste non exhaustive de plantes connues comme possédant de l' α amyrine, de la β amyrine et un mélange d' α, de β amyrine en petites quantités (détectées et isolées) sont répertoriées :

L'α Amyrine a été isolée de la résine de *Boswellia carterii (Burseraceae)* (Wang et al., 2011), détectée dans le bois de tige et l'écorce de *Populus sp (Salicaceae)* (Xu et al., 2010), isolée (65 mg / kg) du n -hexane extrait des feuilles de *Melastoma malabathricum (Melastomataceae)* (Sirat et al., 2010), identifiée dans l'extrait méthanolique de l'écorce de tige de *Poncirus trifoliate (Rutaceae*) (Feng et al., 2010), isolée (1 mg / kg) du méthanol extrait d'écorce de tige de l'arbre africain *Antiaris sp (Moraceae)* (Vouffo et al., 2010), détectée dans l'huile de graines de baies de Saskatoon *Amelanchier alnifolia (Rosaceae)* (Bakowska-Barczak et al., 2009), isolée (23 mg / kg) d'extrait au méthanol d'écorce de tige et de feuilles de *Ficus pandurata (Moraceae)* (Ramadan et al., 2009), de rhizomes séchés de *Nelumbo nucifera (Nelumbonaceae*) (Chaudhuri et al., 2009), et détectés dans le blé panifiable *Triticum aestivum (Poaceae)* (Nurmi et al., 2008 ).

La β amyrine a été isolée et détectée dans divers végétaux : une fraction éthanolique de résine oléo-gomme de *Ferula gummosa (Apiaceae)* (Jalali et al., 2011), la plante *Carpobrotus edulis (Aizoaceae),* (Martins et al., 2011), les feuilles de *Clerodendrum inerme (Verbenaceae)* (22,5 mg / kg) (Parveen et al., 2010), extrait chloroformique des parties aériennes du plantes ou cals *d'Euphorbia tirucalli (Euphorbiaceae)* (Uchida et al., 2010), huile de graines de *Capparis spinosa (Capparaceae)* (Tlili et al., 2011), extrait chloroformique des feuilles de *Ficus benjamina (var. camosa) (Moraceae)* (Simo et al., 2009), feuilles (2 mg / kg) de *Pyrenacantha staudii* (Icacinaceae), (Falodun et al., 2009), extrait à l'éthanol de feuilles de *Olea europaea (Oleaceae)* (Wang et al., 2009), extrait à l'acétate d'éthyle de pelures de pommes de la variété Red Delicious *Malus domestica (Rosaceae)* (He et al., 2008), feuilles séchées à l'air de *Tectona philippinensis (Lamiaceae),* une plante médicinale philippine endémique (Ragasa et al., 2008), un extrait mixte de benzène et de chloroforme de feuilles de *Rhus alata (Anacardiaceae)* (Parveena et al., 2008), et un extrait d'écorce de tige de *Piptadenia africana* ou *Piptadeniastrum africanum (Mimosaceae)* du Cameroun (Mbouangouere et al., 2008).

Un mélange d' α et de β amyrine a été détecté dans les plantes suivantes: des extraits de n-hexane et de chloroforme de la couche de cire épicuticulaire de *Mandevilla guanabarica* et *Mandevilla moricandiana (Apocynaceae)* (Cordeiro et al., 2011), un extrait éthanolique de racines de *Salacia amplifolia (Celastraceae)* (Wang et al., 2011) et des extraits chloroformiques de chèvrefeuille bleu *Lonicera caerulea (Caprifoliaceae)* (Palikova et al., 2008). Par génie génétique et moléculaire, l'expression d'une oxydosqualène cyclase (OSC) et β-amyrine synthase, qui interviennent dans la cyclisation du 2,3-oxydosqualène pour produire de la β-amyrine a donnée avec succès dans *Saccharomyces cerevisiae* de la β-amyrine (Sun R, 2017).

Toujours par génie génétique et moléculaire, la production d'α amyrine est désormais possible chez *Saccharomyces cerevisiae.* En effet, 2 Alpha amyrines synthases α-AS, EjAS et MdOSC1 d'*Eriobotrya japonica* et *Malus* × *domestica* ont été exprimées dans levure *S*. *cerevisiae.* Les levures avec EjAS ont produit de l'α-amyrine et de la β amyrine dans un rapport de 17: 3. Les levures avec MdOSC1 ont produit de l'α-amyrine, de la β amyrine et du lupéol dans un rapport 86: 13: 1 (YU Y, 2018).

De manière inattendue et inédite l'inventeur a découvert que les triterpènes pentacycliques choisis parmi l'α et la β amyrine peuvent être utilisés en tant que principes actifs pour l'inhibition de la PCSK3.

Dans le présent texte, le « triterpène pentacyclique choisi parmi l'α et la β amyrine» utilisé selon l'invention est également appelé « l'inhibiteur de la PCSK3 » et « le principe actif ».

L'inhibition de PCSK et notamment la furine permet d'enrailler bon nombre de phénomème infectieux par inhibition du clivage protéïque qui active la protéine toxique ou le microorganisme pathogène qui la porte. L'activité anti-infectieuse portée par les inhibiteurs de PCSK et notamment la furine n'est donc pas de nature biocide.

L'inhibiteur de furine selon l'invention est un principe actif avantageusement choisi parmi les amyrines purifiées, mais aussi parmi les extraits naturels contenant des amyrines et dérivés d'amyrines et notamment les extraits de Burséracées, et plus particulièrement les extraits de Protium heptaphyllum. Mais plus généralement, dans le cadre de cette invention, les amyrines inhibitrices de l'activité furine qui constituent le nouveau principe actif selon l'invention, peuvent être issues du règne végétal, de plantes capables de produire des terpénoïdes de type triterpènes pentacycliques et plus particulièrement les α et β amyrines et leurs dérivés, de familles aussi diverses que *Aizoaceae, Anacardiaceae, Apiaceae, Apocynaceae, Asclepiadaceae, Bombacaceae, Burseraceae,* Caesalpinaceae, *Capparaceae, Caprifoliaceae,* Celastraceae, Clusiaceae, *Cucurbitaceae, Euphorbiaceae, Fabaceae, lcacinaceae, Irvingiaceae, Lamiaceae, Malpighiaceae, Malvaceae, Melastomataceae, Mimosaceae, Moraceae, Myrsinaceae, Myrtaceae, Nelumbonaceae, Oleaceae, Poaceae, Rosaceae, Rutaceae, Salicaceae, Sapotaceae, Theaceae, Verbenaceae, Vitaceae* ; Plus particulièrement dans le cadre de cette invention, les amyrines inhibitrices de l'activité pourront être issues du règne végétal, de plantes capables de produire des terpénoïdes de type triterpènes pentacycliques et plus particulièrement les α et β amyrines et leurs dérivés, d'espèces aussi diverses que *Acacia sp ou Senegalia visco (Fabaceae), Amelanchier alnifolia (Rosaceae), Amphipterygium adstringens (Anacardiaceae), Antiaris sp (Moraceae), Ardisia elliptica (Myrsinaceae), Bombax ceiba (Bombacaceae), Bombax malabaricum (Bombacaceae), Boswellia carterii (Burseraceae), Bursera glabrifolia (Burseraceae), Bursera simaruba (Burseraceae), Byrsonima crassifolia* ou *crassa (Malpighiaceae), Byrsonima fagifolia (Malpighiaceae), Calotropis gigantea (Asclepiadaceae), Camellia japonica (Theaceae), Capparis spinosa (Capparaceae), Carpobrotus edulis (Aizoaceae), Cassia obtusifolia (Caesalpinaceae), Clerodendrum inerme (Verbenaceae), Commiphora holtziana (syn. Commiphora erythraea) (Burseraceae), Duranta repens (Verbanaceae), Eucalyptus globulus (Myrtaceae), Euphorbia tirucalli (Euphorbiaceae), Ferula gummosa (Apiaceae), Ficus benjamina (Moraceae), Ficus carica (Moraceae), Ficus cordata (Moraceae), Ficus pandurata (Moraceae),* Garcinia subelliptica (Clusiaceae), *Klainedoxa gabonensis (Irvingiaceae), Lonicera caerulea (Caprifoliaceae), Ligustrum sp (Oleaceae), Malus domestica (Rosaceae), Melia azedarach (Meliaceae), Mandevilla guanabarica et Mandevilla moricandiana (Apocynaceae), Melastoma malabathricum (Melastomataceae), Nelumbo nucifera (Nelumbonaceae), Olea europaea (Oleaceae), Piptadeniastrum africanum (Mimosaceae), Poncirus trifoliate (Rutaceae), Populus sp (Salicaceae), Pouteria sp (Sapotaceae), Protium sp (Burseraceae), Pyrenacantha staudii (Icacinaceae), Rhus alata (Anacardiaceae), Salacia amplifolia (Celastraceae), Siraitia grosvenorii (Cucurbitaceae), Tectona philippinensis (Lamiaceae), Triticum aestivum (Poaceae),et Vitis vinifera (Vitaceae).*

Dans le cadre de cette invention, les amyrines inhibitrices de l'activité de la furine qui constituent le nouveau principe actif selon l'invention, pourront également être issues de la biotechnologie, c'est-à-dire produites via des bactéries ou levures, ou bien encore par des enzymes.

Toujours dans le cadre de cette invention, les amyrines inhibitrices de l'activité de la furine qui constituent le nouveau principe actif selon l'invention pourront être issues de tout procédé de chimie organique de synthèse.

Enfin dans le cadre de cette invention, les amyrines inhibitrices de l'activité de la furine qui constituent le nouveau principe actif selon l'invention pourront être issues des extraits naturels issus du règne animal, de préférence extraits chez les arthropodes, notamment chez les insectes du genre Platyphora à partir des espèces chrysomèles.

Pour la mise en œuvre de produits thérapeutiques dans le cadre de l'invention, le principe actif selon l'inventeur peut être préalablement solubilisé ou vectorisé de sorte à pouvoir l'utiliser et le doser dans diverses préparations.

Dans le cadre de la présente invention, les corps gras qui solubilisent ou vectorisent le principe actif peuvent comprendre des huiles, des beurres et des cires hydrocarbonées d'origine animale ou végétale ou encore minérale, des huiles siliconées, ou leur mélange, mais aussi des additifs lipophiles. Comme huiles ou beurres hydrocarbonées, nous pouvons notamment citer: les huiles ou beurres végétaux ou animaux, et plus particulièrement les triglycérides; les éthers de synthèse; les hydrocarbures linéaires ou ramifiés, d'origine minérale ou synthétique tels que la vaseline ; les esters de synthèse tel que le myristate d'isopropyl; les benzoates d'alcools gras; des heptanoates, octanoates, décanoates ou ricinoléates d'alcools ou de polyalcools; les esters hydroxylés comme le lactate d'isostéaryle, les esters de polyols; les alcools gras tel que l'octyldodecanol; les acides gras supérieurs tel que l'acide linolénique; les huiles siliconées de type polyméthylsiloxane et leurs mélanges. Comme cire végétale, nous pouvons notamment citer la cire de carnauba, de candelilla, de jojoba ou tout autre composé végétal constitué d'un ester d'éthylène glycol et de deux acides gras ou d'un monoester d'acide gras et d'alcool à longue chaîne ; comme cire animale, nous pouvons notamment citer la cire d'abeille.

Comme autre corps gras, nous pouvons notamment citer : les huiles essentielles ; les composés aromatiques naturels et de synthèses lipophiles; les vitamines liposolubles naturelles ou synthétiques telle que le tocophérol ou l'alphatocopheryl acétate.

Le principe actif selon l'invention est caractérisé par sa teneur en amyrines. Les amyrines sont soit de l'α amyrine à 100% aussi appelée viminalol, soit de la β amyrine à 100%, mais préférentiellement un mélange d'α et β amyrine. Le mélange préférentiel en amyrines sera entre 20%-80% d'α amyrine et 20%-80% de β amyrine.

Les amyrines en tant que principe actif selon l'invention peuvent être des extraits biotechnologiques, végétaux ou animaux titrés en α et/ou β amyrines.

Les amyrines en tant que principe actif selon l'invention peuvent également être le fruit d'une synthèse chimique organique titrée en α et/ou β amyrines.

Selon l'invention, les amyrines sont significativement actives à partir d'un dosage de 25 ppm, elles sont très actives à 250 ppm et atteignent leur plateau d'activité maximum autour de 1000 ppm. Néanmoins, la toxicité des amyrines étant très faible selon les connaissances actuelles, il est envisageable d'utiliser les amyrines en produits thérapeutiques à forte concentration. Ainsi, les produits thérapeutiques peuvent contenir en tant que principe actif de 10 ppm (0,001%) à 100% d'amyrine.

La présente invention vise aussi un produit thérapeutique comprenant un triterpène pentacyclique, destiné aux mammifères, comprenant de l'amyrine ou dérivés en une teneur comprise entre 0.001% et 100% en poids d'amyrine ou dérivés par rapport au poids total du produit thérapeutique.

Le principe actif selon l'invention se prépare et s'utilise par l'Homme de l'art en préparation thérapeutique, pur ou après une pré-solubilisation dans l'un des composés vecteur ou solvant décrit plus haut.

A titre d'exemple, il est possible de solubiliser les amyrines dans de l'éthanol 96° à environ 55% de sorte à obtenir une solution active liquide homogène facile à doser et diluer.

Un autre exemple consiste à solubiliser les amyrines dans un ester de type ester méthylique de Colza (methyl canolate) jusque 45% de sorte à obtenir une solution active liquide homogène facile à doser et diluer.

Encore un exemple consiste à solubiliser les amyrines dans une huile de tournesol jusque 30% de sorte à obtenir une solution active liquide homogène facile à doser et diluer.

Selon un autre brevet de l'inventeur FR2977169B1, la capacité du principe actif, en tant que composé majoritaire de résine végétale, à créer une interface en milieu aqueux ou polaire entre la phase mobile polaire et la phase dispersée, à savoir son vecteur ou son solvant plutôt apolaire, de sorte à créer une pseudo émulsion où le principe actif se positionne préférentiellement à l'interface de la phase dispersée et dispersante.

Le principe actif selon l'invention peut s'employer par l'homme de l'art, dans une composition thérapeutique de toute polarité sous la forme d'un produit solide de type pastille, suppositoire ou de baume ou cire topique.

Le principe actif selon l'invention peut s'employer dans une composition thérapeutique de toute polarité sous la forme d'un produit liquide de type injectable sous-cutané, injectable intra-veineuse, collyre, spray, sérum ou sirop buvable.

Le principe actif selon l'invention peut s'employer par l'homme de l'art, dans une composition thérapeutique de toute polarité sous la forme d'un produit d'application topique sous forme de lotion, de gel, de sérum, d'émulsion, de patch, d'huile, de solution lavante ou désinfectante.

Le présent texte décrit aussi que le principe actif selon l'invention, en tant qu'inhibiteur de l'activité de la furine, peut s'employer
- dans les milieux de culture biologique, en tant d'additif ou agent de référence ou de contrôle d'un test ou d'un essai ;
- comme d'additif technique ou cible dans un système de diagnostic médical, mais aussi dans un système de dépistage sanguin ou environnemental ;

Le présent texte décrit aussi que, en marge de l'activité thérapeutique, le principe actif selon l'invention, en tant qu'inhibiteur de l'activité de la furine, peut s'employer
- en produit de type spray ou aérosol assainissant environnemental ou pour tout produit phyto-et /ou zoo-technique sous quelque forme que ce soit ;
- par pulvérisation sous une forme liquide pour faire du pelliculage sur des surfaces de sorte à générer une barrière bioactive de type anti-virale. A titre d'exemple non exhaustif, nous pouvons envisager de pulvériser une solution alcoolique d'amyrines ou d'extrait résineux riche en amyrines et dérivés sur des masques faciaux de sorte à augmenter l'effet barrière virale de ces dispositifs, car en effet le solvant alcoolique séchant rapidement, les amyrines sont rapidement transférées et déposées, elles pelliculent alors les surfaces imprégnées.

Les exemples qui suivent visent à illustrer l'invention sans en limiter la portée.

### EXEMPLES

### Exemple 1 Inhibition de l'activité de l'enzyme Furine

L'expérience est réalisée en utilisant une furine humaine recombinante, qui catalyse le clivage d'un substrat spécifique fluorescent.

### Produit de référence

Le décanoyl-Arg-Val-Lys-Arg-CMK à 100 nm a été utilisé comme inhibiteur de référence de l'activité de la furine.

### Protocole d'incubation

La furine est pré-incubée pendant 10 minutes à température ambiante en l'absence (contrôle) ou en présence du produit de référence ou de concentrations croissantes du composé testé :
«Amyrines purifiées de Protium heptaphyllum»; 25 ppm; 250 ppm et 1000 ppm (v / v). Le produit «Amyrines purifiées de Protium heptaphyllum» , AMY à 1000 PPM correspond à une solution 0,5% en volume du produit HTRE commercialisé par la société Ephyla.

À la fin de l'étape de pré-incubation, un substrat de furine a été ajouté et les conditions expérimentales ont été incubées à nouveau à température ambiante à l'abri de la lumière pendant 5 minutes. Toutes les expériences ont été réalisées en *triplicate.*

### Préparation du composé :

Le composé d'essai " Amyrines purifiées de Protium heptaphyllum " pré-solubilisé à 10 000 ppm dans du DMSO. La solution a ensuite été diluée dans du tampon d'essai afin d'atteindre les concentrations décrite ci-dessus.

### Protocole d'évaluation

Le clivage du substrat fluorescent de furine a été suivi pendant 5 minutes après l'addition du substrat en lisant la fluorescence à 485 nm / 535 nm.

### Statistiques

Les résultats sont exprimés en RFU (unités fluorescentes relatives) +/- S.D. (Écart-type).

La signification statistique de la différence observée entre les groupes «Contrôle» et «Produit de référence» a été évaluée par un test t de Student (p <0,001).

La signification statistique de la différence observée entre les groupes «Contrôle» et «Composé test» a été évaluée par une analyse ANOVA unidirectionnelle, suivie d'un test HolmSidak ( p <0,05).

### Résultats

Le composé «Amyrines purifiées de Protium heptaphyllum », désigné comme « AMY » (ci-dessous) dans les conditions expérimentales choisies inhibe de manière significative l'activité de la furine:

| | Activité de la furine (en% de contrôle) |
|---|---|
| «AMY» à 25 ppm (v / v) | 84,6% (p <0,001) |
| «AMY» à 250 ppm (v / v) | 12,0% (p <0,001) |
| «AMY» à 1000 ppm (v / v) | 0,7% (p <0,001) |

### Produit et véhicule de référence

L'inhibiteur de référence de la furine nommé Decanoyl-Arg-Val-Lys-Arg-CMK, testé à 100 nm, a significativement inhibé l'activité de la furine de 97,9% (p <0,001).

Ce résultat était attendu et valide l'étude.

### Conclusion

À 250 ppm, «Amyrines purifiées de Protium heptaphyllum» inhibe 88% de l'activité de l'enzyme Furine

A 1000 ppm, «Amyrines purifiées de Protium heptaphyllum» inhibe 99,3% de l'activité de l'enzyme Furine.

### Exemple 2 : Effet anti-infectieux contre un virus à couronne SARS COV2

L'expérience a été réalisée en utilisant une solution à 0,5% de HTRE qui correspond au complexe AMY à 1000 PPM décrit dans l'exemple 1 ci-dessus.

Le test utilise 2 composants principaux.

D'une part, une souche cellulaire Humaine HEK293 recombinante exprimant ACE2 humain de pleine longueur (Genbank # NM_021804.3), avec une expression de surface de ACE2 confirmée par cytométrie en flux.

D'autre part, les lentivirus pseudotypés SARS-CoV-2 Spike produits avec SARS-CoV-2 Spike (numéro d'accès Genbank QHD43416.1) comme glycoprotéines d'enveloppe au lieu du VSV-G couramment utilisé. Ces pseudovirions contiennent également le gène de la luciférase de luciole dirigé par un promoteur du CMV, par conséquent, l'entrée cellulaire médiée par le pic peut être mesurée de manière pratique via l'activité de rapporteur de la luciférase.

### Protocole :

Étape 1: les cellules ACE2-HEK sont mises en puits de culture. A cet effet, les cellules ACE2-HEK sont décongelées dans le milieu de décongélation 1, amplifiées dans du milieu de croissance 1N, puis récoltées et mise en culture dans des plaques de culture à 96 puits à fond plat blanc et transparent à 10.000 cellules / puits dans 50 µl de milieu de décongélation 1. Les cellules sont incubées pendant une nuit à 37 ° C.

### Étape 2: Test d'infection lentivirale pseudotypée

Le jour suivant, le contrôle visuel de l'homogénéité et de l'intégrité de la couche cellulaire est validé à l'aide d'un microscope inversé et les ingrédients de test suivants sont préparés :
Trois solution de HTRE ont été préparées C1 ( 0.5%), C2 ( 0.25%) et C3 ( 0.1%) ; puis ces solutions ont été mélangées avec du DMSO selon le rapport 75% de solution de HTRE et 25 % de DMSO.

Lors de l'addition de DMSO, HTRE forme 2 phases de volume similaire appelées « up » (haut) et «down» (bas). La phase «down» affiche une couleur jaune marquée. HTRE «up» est testé aux 3 concentrations 0.5 (C1), 0.25 (C2) et 0.1% (C3), tandis que HTRE «down» est testé à deux concentrations, C1 et C2. La concentration finale de DMSO dans les puits d'analyse correspondants est de 0,5%.

Les extraits sont dilués à une concentration intermédiaire 11x dans le milieu de décongélation 1, pour un transfert ultérieur de 5 µl dans la plaque de dosage. Après une période d'incubation de 30 min à 37 ° C, 5 pl de lentivirus pseudotypé non dilué (Bald ou S1-Spike) sont ajoutés aux puits d'analyse correspondants. Comme contrôles négatifs supplémentaires, 5 µl de milieu de décongélation 1 contenant soit 5,5% de DMSO ou PBS sont utilisés. En tant que contrôle positif, le mAb bloquant ACE2 est utilisé à une concentration finale de 0,5 µM dans les puits d'analyse.

Le bald pseudo virion est un témoin additionnel (virus sans couronne « Spike protéines ») qui montre que sans Spike protéines le virus Bald n'a pas la capacité d'infecter les cellules.

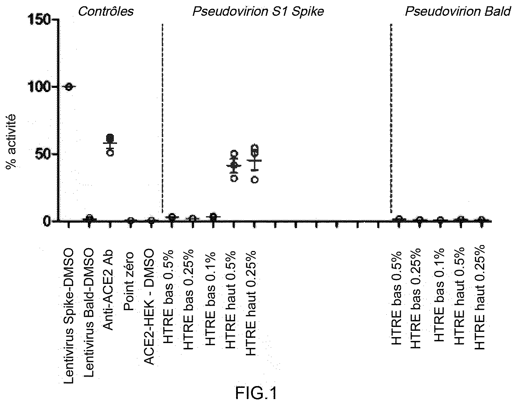

Les résultats obtenus sont présentés dans la figure 1 annexée.

### Conclusion

- Bald vs Spike: augmentation supérieure à 57 fois du signal RLU lors d'une infection au lentivirus pseudotypé S1 Spike, validant l'expérience.
- Spike vs anti-ACE2 Spike: Chute de 41,91% du signal RLU induite par l'anticorps bloquant utilisé à des concentrations proches des valeurs IC50 mesurées par le fournisseur du kit, validant l'expérience.
- HTRE «down»: Chute de 96,93% du signal RLU induite par l'anticorps bloquant utilisé à la concentration 0,5%; Chute de 98% du signal RLU induite par l'anticorps bloquant utilisé à la concentration 0,25%; Chute de 96,62% du signal RLU induite par l'anticorps bloquant utilisé à la concentration de 0,1%
   L'effet anti-infectieux contre un virus à couronne SARS COV2 est ainsi démontré et probant dès la dose de 0,1% de HTRE (soit 200 PPM AMY équivalent) avec une réduction de 96,62% de l'infection virale.
- HTRE «up»: Chute de 58,66% du signal RLU induite par l'anticorps bloquant utilisé à la concentration 0,5%; Chute de 54,58% du signal RLU induite par l'anticorps bloquant utilisé à la concentration 0,25%. La fraction HTRE «up» est simplement du HTRE moins bien solubilisé dans le milieu car très peu liée au DMSO, cette moins bonne solubilisation se traduit de manière attendue par un score significatif mais moins probant.

### BIBLIOGRAPHIE

Braun E et al., 2019 : « Furin-mediated protein processing in infectious diseases and cancer » Elisabeth Braun & Daniel Sauter, Clinical & Translational Immunology 2019; 8: e1073.
Sauf mention précise, les références de tous les articles cités dans le présent texte sont présentées en fin de l'article Braun E et al., 2019 cité ci-dessus.

## Revendications

1. Triterpène pentacyclique choisi parmi les α et β amyrines, pour son utilisation en tant que principe actif inhibiteur de la PCSK3 aussi appelée furine, pour le traitement thérapeutique de maladies infectieuses chez les mammifères pour bloquer les toxines microbiologiques de type AB et les maintenir passives et sans virulence, pour stopper l'anthrax, comme anti-viral chez les virus enveloppés bloquant l'infection et la pathogénécité des Coronavirus, des Flavivirus, des Togavirus, des Bornavirus, des Bunyavirus, des Filovirus, des Paramyxovirus, des Pneumovirus et Retroviridae, et plus particulièrement du Virus d'Immunodéficience Humaine (VIH), du virus du sarcome de Rous, des virus de la leucémie murine, du virus de Marburg, des cinq espèces d'Ebolavirus, du virus de l'encéphalite à tiques et des virus de la dengue, pour favoriser le bon fonctionnement de la réaction immunitaire de défense de l'hôte chez les virus de la dengue, comme anti-viral chez les virus non enveloppés en bloquant la propagation infectieuse des papillomavirus.

2. Triterpène pentacyclique pour son utilisation en tant que principe actif selon la revendication 1, **caractérisé en ce que** ledit triterpène pentacyclique est choisi parmi les amyrines purifiées et les extraits naturels contenant des amyrines, notamment les extraits de Burséracées, et plus particulièrement les extraits de Protium heptaphyllum.

3. Triterpène pentacyclique pour son utilisation en tant que principe actif selon la revendication 2 , **caractérisé en ce que** les extraits naturels sont issus de plantes choisies parmi *Acacia sp* ou *Senegalia visco (Fabaceae), Amelanchier alnifolia (Rosaceae), Amphipterygium adstringens (Anacardiaceae), Antiaris sp (Moraceae), Ardisia elliptica (Myrsinaceae), Bombax ceiba (Bombacaceae), Bombax malabaricum (Bombacaceae), Boswellia carterii (Burseraceae), Bursera glabrifolia (Burseraceae), Bursera simaruba (Burseraceae), Byrsonima crassifolia* ou *crassa (Malpighiaceae), Byrsonima fagifolia (Malpighiaceae), Calotropis gigantea (Asclepiadaceae), Camellia japonica (Theaceae), Capparis spinosa (Capparaceae), Carpobrotus edulis (Aizoaceae), Cassia obtusifolia (Caesalpinaceae), Clerodendrum inerme (Verbenaceae), Commiphora holtziana (syn. Commiphora erythraea) (Burseraceae), Duranta repens (Verbanaceae), Eucalyptus globulus (Myrtaceae), Euphorbia tirucalli (Euphorbiaceae), Ferula gummosa (Apiaceae), Ficus benjamina (Moraceae), Ficus carica (Moraceae), Ficus cordata (Moraceae), Ficus pandurata (Moraceae), Garcinia subelliptica (Clusiaceae), Klainedoxa gabonensis (Irvingiaceae), Lonicera caerulea (Caprifoliaceae), Ligustrum sp (Oleaceae), Malus domestica (Rosaceae), Melia azedarach (Meliaceae), Mandevilla guanabarica* et *Mandevilla moricandiana (Apocynaceae), Melastoma malabathricum (Melastomataceae), Nelumbo nucifera (Nelumbonaceae), Olea europaea (Oleaceae), Piptadeniastrum africanum (Mimosaceae), Poncirus trifoliate (Rutaceae), Populus sp (Salicaceae), Pouteria sp (Sapotaceae), Protium sp (Burseraceae), Pyrenacantha staudii (Icacinaceae), Rhus alata (Anacardiaceae), Salacia amplifolia (Celastraceae), Siraitia grosvenorii (Cucurbitaceae), Tectona philippinensis (Lamiaceae), Triticum aestivum (Poaceae),et Vitis vinifera (Vitaceae).*

4. Triterpène pentacyclique pour son utilisation en tant que principe actif selon la revendication 2, **caractérisé en ce que** les extraits naturels sont issus du règne animal, de préférence les arthropodes, notamment chez les insectes du genre Platyphora à partir des espèces chrysomèles.

5. Triterpène pentacyclique pour son utilisation en tant que principe actif selon la revendication 1, **caractérisé en ce que** ledit triterpène pentacyclique est choisi parmi les amyrines issues de la biotechnologie, c'est-à-dire produites via des bactéries ou levures, ou bien encore par des enzymes.

6. Triterpène pentacyclique pour son utilisation en tant que principe actif selon la revendication 1, **caractérisé en ce que** ledit triterpène pentacyclique est choisi parmi les amyrines issues d'un procédé de synthèse en chimie organique.

7. Triterpène pentacyclique pour son utilisation en tant que principe actif selon l'une quelconque des revendications précédentes, **caractérisé en ce que** ledit triterpène pentacyclique est constitué d'α amyrine (à 100% ou viminalol), ou de β amyrine (à 100%), ou d'un mélange d'α et β amyrines contenant de 20% à 80% d'α amyrine et de 20% à 80% de β amyrine de préférence d'un mélange d'α et β amyrines contenant de 20% à 80% d'α amyrine et de 20% à 80% de β amyrine

8. Produit thérapeutique comprenant au moins un triterpène pentacyclique pour son utilisation selon l'une quelconque des revendications 1 à 7, destiné aux mammifères, **caractérisé en ce qu'**il comprend de l'amyrine en une teneur comprise entre 0.001% et 100% en poids d'amyrine par rapport au poids total du produit thérapeutique.

9. Produit thérapeutique comprenant au moins un triterpène pentacyclique pour son utilisation selon l'une quelconque des revendications 1 à 8 destiné aux mammifères, **caractérisé en ce qu'**il présente la forme d'un produit liquide de toute polarité type injectable sous-cutané, injectable intra-veineuse, collyre, spray, sérum ou sirop buvable.

10. Produit thérapeutique comprenant au moins un triterpène pentacyclique pour son utilisation selon l'une quelconque des revendications 1 à 8 destiné aux mammifères, **caractérisé en ce qu'**il présente la forme d'un produit solide de type pastille, suppositoire ou de baume ou cire topique.

11. Produit thérapeutique comprenant au moins un triterpène pentacyclique pour son utilisation selon l'une quelconque des revendications 1 à 8 destiné aux mammifères, **caractérisé en ce qu'**il présente la forme d'un produit d'application topique tel que lotion, gel, sérum, émulsion, patch, huile, solution lavante ou désinfectante.

## Patentansprüche

1. Pentacyclisches Triterpen, ausgewählt aus α- und β-Amyrinen aufgrund seiner Wirkung als Inhibitor von PCSK3, auch Furin genannt, zur Therapie von Infektionskrankheiten bei Säugetieren, um mikrobielle AB-Toxine zu blockieren und sie inaktiv und avirulent zu halten, zur Bekämpfung von Anthrax, als Virostatikum bei behüllten Viren, zur Blockierung der Infektion und Pathogenität von Coronaviren, Flaviviren, Togaviren, Bornaviren, Bunyaviren, Filoviren, Paramyxoviren, Pneumoviren und Retroviren, und insbesondere des Humanen Immundefizienz-Virus (HIV), des Rous-Sarkom-Virus, von Maus-Leukämie-Viren, des Marburg-Virus, der fünf Ebolavirus-Arten, des FSME-Virus und von Dengue-Viren, um die Immunantwort des Wirts gegenüber Dengue-Viren zu fördern, als Virostatikum bei unbehüllten Viren, wo es die infektiöse Ausbreitung von Papillomaviren blockiert.

2. Pentacyclisches Triterpen zur Verwendung als Wirkstoff nach Anspruch 1, **dadurch gekennzeichnet, dass** das pentacyclische Triterpen unter gereinigten Amyrinen und natürlichen, Amyrin enthaltenden Extrakten, nämlich Burseraceae-Extrakten und insbesondere Protium heptaphyllum-Extrakten, ausgewählt wird.

3. Pentacyclisches Triterpen zur Verwendung als Wirkstoff nach Anspruch 2, **dadurch gekennzeichnet, dass** die natürlichen Extrakte von ausgewählten Pflanzen stammen, darunter *Acacia sp oder Senegalia visco (Fabaceae), Amelanchier alnifolia (Rosaceae), Amphipterygium adstringens (Anacardiaceae), Antiaris sp (Moraceae), Ardisia elliptica (Myrsinaceae), Bombax ceiba (Bombacaceae), Bombax malabaricum (Bombacaceae), Boswellia carterii (Burseraceae), Bursera glabrifolia (Burseraceae), Bursera simaruba (Burseraceae), Byrsonima crassifolia oder crassa (Malpighiaceae), Byrsonima fagifolia (Malpighiaceae), Calotropis gigantea (Asclepiadaceae), Camellia japonica (Theaceae), Capparis spinosa (Capparaceae), Carpobrotus edulis (Aizoaceae), Cassia obtusifolia (Caesalpinaceae), Clerodendrum inerme (Verbenaceae), Commiphora holtziana (syn. Commiphora erythraea) (Burseraceae), Duranta repens (Verbanaceae), Eucalyptus globulus (Myrtaceae), Euphorbia tirucalli (Euphorbiaceae), Ferula gummosa (Apiaceae), Ficus benjamina (Moraceae), Ficus carica (Moraceae), Ficus cordata (Moraceae), Ficus pandurata (Moraceae), Garcinia subelliptica (Clusiaceae), Klainedoxa gabonensis (Irvingiaceae), Lonicera caerulea (Caprifoliaceae), Ligustrum sp (Oleaceae), Malus domestica (Rosaceae), Melia azedarach (Meliaceae), Mandevilla guanabarica und Mandevilla moricandiana (Apocynaceae), Melastoma malabathricum (Melastomataceae), Nelumbo nucifera (Nelumbonaceae), Olea europaea (Oleaceae), Piptadeniastrum africanum (Mimosaceae), Poncirus trifoliate (Rutaceae), Populus sp (Salicaceae), Pouteria sp (Sapotaceae), Protium sp (Burseraceae), Pyrenacantha staudii (Icacinaceae), Rhus alata (Anacardiaceae), Salacia amplifolia (Celastraceae), Siraitia grosvenorii (Cucurbitaceae), Tectona philippinensis (Lamiaceae), Triticum aestivum (Poaceae), und Vitis vinifera (Vitaceae).*

4. Pentacyclisches Triterpen zur Verwendung als Wirkstoff nach Anspruch 2, **dadurch gekennzeichnet, dass** die natürlichen Extrakte aus dem Tierreich stammen, vorzugsweise aus Arthropoden, und zwar aus Insekten der Gattung Platyphora und der Species der Chrysomelidae.

5. Pentacyclisches Triterpen zur Verwendung als Wirkstoff nach Anspruch 1, **dadurch gekennzeichnet, dass** das pentacyclische Triterpen unter biotechnologisch gewonnenen, d. h. mithilfe von Bakterien oder Hefen oder auch Enzymen hergestellten Amyrinen, ausgewählt wird.

6. Pentacyclisches Triterpen zur Verwendung als Wirkstoff nach Anspruch 1, **dadurch gekennzeichnet, dass** das genannte pentacyclische Triterpen unter Amyrinen ausgewählt wird, die mittels eines Verfahrens der organischen Chemie synthetisiert wurden.

7. Pentacyclisches Triterpen zur Verwendung als Wirkstoff nach einem der vorstehenden Ansprüche, **dadurch gekennzeichnet, dass** das pentacyclische Triterpen aus α-Amyrin bzw. Viminalol (zu 100 %) oder aus β-Amyrin (zu 100%) oder aus einer Mischung aus α- und β-Amyrinen besteht, die 20 % bis 80 % α-Amyrin und 20 % bis 80 % β-Amyrin enthält, vorzugsweise eine Mischung aus α- und β-Amyrinen mit 20 % bis 80 % α-Amyrin und 20 % bis 80 % β-Amyrin.

8. Therapeutisches Produkt, das mindestens ein pentacyclisches Triterpen zur Verwendung nach einem der Ansprüche 1 bis 7 enthält, das für Säugetiere bestimmt ist, **dadurch gekennzeichnet, dass** sein Gehalt an Amyrin zwischen 0,001 % und 100 % seines Gesamtgewichts ausmacht.

9. Therapeutisches Produkt, das mindestens ein pentacyclisches Triterpen zur Verwendung nach einem der Ansprüche 1 bis 8 umfasst, das für Säugetiere bestimmt ist, **dadurch gekennzeichnet, dass** es in Form einer Flüssigkeit mit beliebiger Polarität vorliegt, die subkutan injizierbar, intravenös injizierbar, als Augentropfen, Spray, Serum oder trinkbarer Sirup anwendbar ist.

10. Therapeutisches Produkt, das mindestens ein pentacyclisches Triterpen zur Verwendung nach einem der Ansprüche 1 bis 8 umfasst, das für Säugetiere bestimmt ist, **dadurch gekennzeichnet, dass** es in fester Form als Tablette, Zäpfchen, Balsam oder als Wachs zur topischen Anwendung vorliegt.

11. Therapeutisches Produkt, das mindestens ein pentacyclisches Triterpen zur Verwendung nach einem der Ansprüche 1 bis 8 enthält, das für Säugetiere bestimmt ist, **dadurch gekennzeichnet, dass** es in Form eines Produkts zur topischen Anwendung vorliegt, wie beispielsweise als Lotion, Gel, Serum, Emulsion, Pflaster, Öl, Wasch- oder Desinfektionslösung.

## Claims

1. A pentacyclic triterpene selected among α and β amyrins for use as a PCSK3-inhibiting active substance, also called furin, for the therapeutical treatment of infectious diseases among mammals, to inhibit AB-type microbiological toxins and keep them passive and without virulence, to stop anthrax, as an antiviral drug against enveloped viruses, hence inhibiting the infection and pathogenicity of Coronaviruses, Flaviviruses, Togaviridae, Bornaviridae, Bunyaviruses, Filoviridae, Paramyxoviridae, Pneumoviruses and Retroviridae, in particular of the Human Immunodeficiency Virus (HIV), of the Rous sarcoma virus, of the virus murine leukemia viruses, of the Marburg virus, of the five species of ebolavirus, of the tick-borne encephalitis virus and of the dengue viruses, to improve the function of the defensive immune response of the host for the dengue viruses, as an antiviral drug against naked viruses by inhibiting the infectious spread of the papillomaviruses.

2. A pentacyclic triterpene for use as an active substance according to claim 1, **characterized in that** said pentacyclic triterpene is selected among purified amyrins and natural extracts containing amyrins, in particular extracts of Burseraceae, more specifically extracts of Protium heptaphyllum.

3. A pentacyclic triterpene for use as an active substance according to claim 2, **characterized in that** the natural extracts are plant extracts selected among *Acacia sp* or *Senegalia visco (Fabaceae), Amelanchier alnifolia (Rosaceae), Amphipterygium adstringens (Anacardiaceae), Antiaris sp (Moraceae), Ardisia elliptica (Myrsinaceae), Bombax ceiba (Bombacaceae), Bombax malabaricum (Bombacaceae), Boswellia carterii (Burseraceae), Bursera glabrifolia (Burseraceae), Bursera simaruba (Burseraceae), Byrsonima crassifolia or crassa (Malpighiaceae), Byrsonima fagifolia (Malpighiaceae), Calotropis gigantea (Asclepiadaceae), Camellia japonica (Theaceae), Capparis spinosa (Capparaceae), Carpobrotus edulis (Aizoaceae), Cassia obtusifolia (Caesalpinaceae), Clerodendrum inerme (Verbenaceae), Commiphora holtziana (syn. Commiphora erythraea) (Burseraceae), Duranta repens (Verbanaceae), Eucalyptus globulus (Myrtaceae), Euphorbia tirucalli (Euphorbiaceae), Ferula gummosa (Apiaceae), Ficus benjamina (Moraceae), Ficus carica (Moraceae), Ficus cordata (Moraceae), Ficus pandurata (Moraceae), Garcinia subelliptica (Clusiaceae), Klainedoxa gabonensis (Irvingiaceae), Lonicera caerulea (Caprifoliaceae), Ligustrum sp (Oleaceae), Malus domestica (Rosaceae), Melia azedarach (Meliaceae), Mandevilla guanabarica and Mandevilla moricandiana (Apocynaceae), Melastoma malabathricum (Melastomataceae), Nelumbo nucifera (Nelumbonaceae), Olea europaea (Oleaceae), Piptadeniastrum africanum (Mimosaceae), Poncirus trifoliate (Rutaceae), Populus sp (Salicaceae), Pouteria sp (Sapotaceae), Protium sp (Burseraceae), Pyrenacantha staudii (Icacinaceae), Rhus alata (Anacardiaceae), Salacia amplifolia (Celastraceae), Siraitia grosvenorii (Cucurbitaceae), Tectona philippinensis (Lamiaceae), Triticum aestivum (Poaceae), and Vitis vinifera (Vitaceae).*

4. A pentacyclic triterpene for use as an active substance according to claim 2, **characterized in that** the natural extracts are animal extracts, preferably from arthropods, in particular from insects of the genus Platyphora of the chrysomelidae species.

5. A pentacyclic triterpene for use as an active substance according to claim 1, **characterized in that** said pentacyclic triterpene is selected among biotechnological amyrins, which means produced from bacteria or yeasts, or from enzymes.

6. A pentacyclic triterpene for use as an active substance according to claim 1, **characterized in that** said pentacyclic triterpene is selected among amyrins from organic synthesis processes.

7. A pentacyclic triterpene for use as an active substance according to any one of previous claims, **characterized in that** said pentacyclic triterpene is made of α amyrin (100 % or viminalol), or of β amyrin (100 %), or of a mix of α and β amyrins comprising 20 % to 80 % of α amyrin and 20 % to 80 % of β amyrin, preferably of a mix of α and β amyrins comprising 20 % to 80 % of α amyrin and 20 % to 80 % of β amyrin.

8. A therapeutical product comprising at least one pentacyclic triterpene for use according to any one of claims 1 to 7 on mammals, **characterized in that** it comprises between 0.001 % and 100 % amyrin in weight of amyrin relatively to the total weight of the therapeutical product.

9. A therapeutical product comprising at least one pentacyclic triterpene for use according to any one of claims 1 to 8 on mammals, **characterized in that** it is in form of a liquid product of any polarity, such as subcutaneously injectable, intravenously injectable, eye drops, spray, serum or drinking syrup.

10. A therapeutical product comprising at least one pentacyclic triterpene for use according to any one of claims 1 to 8 on mammals, **characterized in that** it is in form of a solid product such as a lozenge, a suppository, a balm or a topic wax.

11. A therapeutical product comprising at least one pentacyclic triterpene for use according to any one of claims 1 to 8 on mammals, **characterized in that** it is in form of a topic application product, such as a lotion, a gel, a serum, an emulsion, a patch, an oil, a cleaning or disinfectant solution.
